# EUROPÄISCHE PATENTSCHRIFT

(11) **EP 2 804 663 B1**
(45) Veröffentlichungstag und Bekanntmachung des Hinweises auf die Patenterteilung: **27.03.2019**
(21) Anmeldenummer: 13710970.8
(22) Anmeldetag: 16.01.2013
(51) Int. Cl.: A61N 1/32, A61N 1/04, A61H 7/00, A61H 23/02

(54) **IN EINER HAND HALTBARES GERÄT ZUR ELEKTRISCH UNTERSTÜTZTEN HAUTBEHANDLUNG**
HAND-HELD DEVICE FOR ELECTRICALLY ASSISTED DERMAL TREATMENT
APPAREIL POUVANT ÊTRE TENU D'UNE SEULE MAIN, DESTINÉ À UN TRAITEMENT DERMATOLOGIQUE REPOSANT SUR L'UTILISATION D'ÉLECTRICITÉ

(30) Priorität: 16.01.2012 DE 102012000563; 14.05.2012 DE 102012009514
(43) Veröffentlichungstag der Anmeldung: 26.11.2014
(73) Patentinhaber: Swiss SPA System Ltd., Hong Kong (CN)
(72) Erfinder: DOYLE, Jr., James N., Ft Myers, Florida 33912 (US); GIMELLI, Bruno, 3052 Zollikofen (CH)
(74) Vertreter: Hebing, Norbert
(86) Internationale Anmeldenummer: PCT/EP2013/000112
(87) Internationale Veröffentlichungsnummer: WO 2013/107635

(56) Entgegenhaltungen:
- EP-A1- 2 384 707
- EP-A2- 2 277 585
- WO-A1-2005/087308
- US-B1- 6 385 487

## Beschreibung

Die Erfindung bezieht sich auf ein in einer Hand haltbares Gerät zur elektrisch unterstützten Hautbehandlung, aufweisend:
Eine erste äußere Elektrode, die in Kontakt zur Hand steht, wenn das Gerät vom Nutzer zum Gebrauch in der Hand gehalten wird, eine zweite äußere Elektrode, die auf den zu behandelnden Hautbereich aufsetzbar ist, und eine elektrische Energiequelle, deren Pole beim Betrieb des Gerätes mit den Elektroden in einer elektrischen Verbindung stehen. Außerdem ist eine Kappe zum lösbaren Aufstecken auf den Basiskörper, die die zweite äußere Elektrode aufweist, und ein elektrisch betriebener vibrator vorgesehen.

Ein Gerät gemäß dem Oberbegriff des Anspruches 1, allerdings ohne Vibrator, ist in der WO 2005/087308 A1 beschrieben. Um das Gerät einzusetzen, nimmt der Nutzer es in die Hand und setzt eine als Elektrode fungierende Kappe auf die zu behandelnden Hautbereiche auf. Mit der ersten Elektrode wird ein elektrisch leitender Kontakt zur Hand und mit der zweiten Elektrode ein elektrisch leitender Kontakt zu dem zu behandelnden Hautbereich hergestellt. Da die beiden Elektroden an je einem Pol einer elektrischen Energiequelle angeschlossen sind, entsteht ein elektrischer Stromkreis, der den Körper des Nutzers mit einschließt, wobei je nach Polung ein positiver oder ein negativer elektrischer Strom von der Kappe in den zu behandelnden Hautbereich einfließt.

Dies kann dazu benutzt werden, die Wirkungsweise von Behandlungscremes bzw. Reinigungscremes zu erhöhen, da die Wirkstoffe einer Behandlungscreme mit dem elektrischen Strom in die Haut transportiert werden und bei einer entgegengesetzten Polung Schmutzstoffe aus der Haut in eine Reinigungscreme transportiert werden.

Das Gerät ist weiterhin mit einer Vielzahl von auswechselbaren Kappen versehen, deren Form jeweils an die zu behandelnde Hautoberfläche angepasst ist. Es gibt eine erste Kappe mit einer relativ glatten Oberfläche, eine zweite Kappe, die kammartig gestaltet ist, die vor allem zur Behandlung der Kopfhaut eingesetzt werden kann, und eine dritte Kappe mit einer welligen Struktur, womit ein Massageeffekt verbunden ist. Mit dem Gerät werden gute Ergebnisse sowohl bei der Hautreinigung als auch bei der Einbringung von Wirkstoffen erzielt. Dies soll aber noch weiter effektiviert werden.

Mit der Erfindung soll die Wirkungsweise des Gerätes gemäß der WO 2005/087308 A1 noch weiter verbessert werden.

Dazu wird in oder an dem Gerät ein elektrisch betriebener Vibrator angebracht.

Ein solcher Vibrator setzt das Gerät in eine schwingende Bewegung, so dass beim Betrieb des Gerätes ein verstärkter Massageeffekt entsteht, wodurch die Hautporen geweitet werden und ein noch besserer Übergang der Schmutzstoffe aus der Haut bzw. der Wirkstoffe in die Haut realisiert wird.

Bezogen auf die US 6, 385, 487 B1 besteht die Aufgabe der Erfindung darin, bei Geräten mit einer großflächigen zweiten äußeren Elektrode einen ausreichend großen Massageeffekt zu erzeugen.

Bei einem solchen Gerät wird der elektrisch betriebene Vibrator in oder an der Kappe angebracht und damit unmittelbar bei der zweiten Elektrode, wobei er derart ausgebildet ist, dass der Bereich des Gerätes besonders stark vibriert, der sich an dem zu behandelnden Hautbereich befindet, so dass beim Betrieb des Gerätes ein verstärkter Massageeffekt auf der zu behandelnden Haut entsteht, wodurch die Hautporen geweitet werden.

Dabei sind Kontaktmittel an der Kappe und an dem Basiskörper, die zur Herstellung einer elektrischen Verbindung zwischen der zweiten Elektrode und der elektrischen Energiequelle dienen, so ausgebildet und angeordnet, dass sie in Kontakt gebracht werden, wenn die Kappe auf den Basiskörper aufgesteckt wird, wobei der Vibrator mit der elektrischen Energiequelle im Basiskörper in einer schaltbaren elektrisch leitenden Verbindung steht.

Bei der Energiequelle kann es sich um Batterien oder auch um Akkus handeln, die in ein Fach des Basiskörpers eingelegt werden. Soweit für das Gerät Akkus vorgesehen sein sollen, ist es denkbar, zu dem Gerät auch ein Ladegerät zu liefern, mit dem die Akkus im Gerät aufgeladen werden können.

Da die Energiequelle im Basiskörper, der Vibrator und eine Elektrode aber in bzw. an der Kappe angeordnet sind, ist eine elektrische Verbindung herzustellen. Die Erfindung sieht dazu vor, dass zwischen dem Basiskörper und der Kappe ein erster und ein zweiter elektrischer Kontakt vorhanden sind, die jeweils aus einem Basiskontaktelement und einem Kappenkontaktelement bestehen, wobei die Basiskontaktelemente im Basiskörper und die Kappenkontaktelemente in der Kappe derart angeordnet sind, dass die Kontakte geschlossen sind, wenn die Kappe auf den Basiskörper aufgesetzt ist, wobei die elektrische Verbindung zwischen der Energiequelle und der zweiten Elektrode über den ersten Kontakt hergestellt ist und wobei der Stromkreis zur Energieversorgung des Vibrators über den ersten und den zweiten Kontakt verläuft.

Da der erste Kontakt sowohl für die Versorgung der zweiten Elektrode als auch für den Anschluss des Vibrators genutzt wird, werden lediglich zwei Kontakte benötigt, obwohl insgesamt drei Anschlüsse (einer zur Elektrode, zwei zum Antrieb des Vibrators) hergestellt werden.

Vorzugsweise bestehen die Kontaktelemente aus Kontaktstiften, deren Stirnseiten bei aufgesteckter Kappe in Kontakt zueinander treten, wobei jeweils ein Kontaktelement eines Kontaktes in axialer Richtung federnd gelagert ist. Dabei ist die Kappe mittels einer lösbaren Raste am Basiskörper gehalten.

Beim Aufsetzen der Kappe drücken die Stifte mit ihren Stirnseiten aufeinander, so dass ein elektrischer Kontakt entsteht. Gleichzeitig wird durch die Vorspannung der Feder eine Federkraft auf die Kappe ausgeübt, so dass die Kappe vom Basiskörper federnd weggedrückt wird und sich leicht vom Basiskörper löst, sobald die Raste, die die Kappe am Basiskörper hält, gelöst wird.

Da der zweite Kontakt lediglich der Stromversorgung des Vibrators dient, ist das Kontaktelement in der Kappe isoliert gegenüber der zweiten Elektrode einzusetzen. Dieses ist insbesondere dann notwendig, wenn die Kappe als Ganzes oder eine äußere Schicht der Kappe aus einem elektrisch leitfähigen Material besteht, wobei das Kappenkontaktelement des ersten elektrischen Kontaktes mit dem Material in elektrisch leitfähiger Weise in Verbindung steht und wobei das Kappenkontaktelement des zweiten elektrischen Kontaktes gegenüber dem leitfähigen Material elektrisch isoliert ist.

Um die zweite Elektrode zu realisieren, ist vorgesehen, dass die Kappe als Ganzes oder eine äußere Schicht der Kappe aus einem elektrisch leitfähigen Material besteht, dass das Kappenkontaktelement des ersten elektrischen Kontaktes mit dem Material in elektrisch leitfähiger Weise in Verbindung steht und dass das Kappenkontaktelement des zweiten elektrischen Kontaktes gegenüber dem leitfähigen Material elektrisch isoliert ist.

Da - wie oben erläutert - das Gerät vorsieht, dass die Polarität der Elektroden austauschbar ist, hat dies zur Folge, dass auch die Polarität der Stromversorgung des Vibrators sich umkehrt. Daher muss der Vibrator einen elektrischen Antrieb besitzen, der unempfindlich ist gegenüber einer Vertauschung der Polarität. Im einfachsten Fall handelt es sich bei dem Vibrator um einen elektrischen Motor, an dessen Welle ein Excenter befestigt ist. Denkbar wären aber auch Schwingungserreger, bei denen eine Masse durch Elektromagnete in schneller Folge mal in die eine, mal in die andere Richtung gezogen wird.

Der elektrische Motor, an dessen Welle ein Excenter befestigt ist, kann in unterschiedlichen Ausrichtungen in der Kappe des Gerätes angeordnet werden. Die Kappe hat an ihrem unteren Ende einen Aufsteckbereich, der auf den Basiskörper aufsteckbar ist, und bildet an ihrem oberen Ende die zweite äußere Elektrode. Damit die Elektrode auf der Haut hin und her schwingt und damit die jeweilige Creme einmassiert wird, ist vorgesehen, dass die Achse des elektrischen Motors und die Welle des elektrischen Motors im Wesentlichen parallel zu einer gedachten Linie zwischen dem Aufsteckbereich und der zweiten äußeren Elektrode angeordnet sind.

Alternativ dazu können die Achse des elektrischen Motors und die Welle des elektrischen Motors im Wesentlichen quer zu einer gedachten Linie zwischen dem Aufsteckbereich und der zweiten äußeren Elektrode angeordnet werden. Auf diese Weise wird ein wechselnder Druck auf die Haut erzeugt, wodurch die jeweilige Creme in die Haut gedrückt wird.

Die Integration des Vibrators in die Kappe soll möglichst in der Weise erfolgen, dass Bauelemente des bisher bekannten Gerätes möglichst weitgehend unverändert beibehalten werden können. Die Erfindung sieht daher vor, dass in der Kappe ein Boden eingesetzt ist, der an seiner der Kappe zugewandten Oberseite einen Halter für den Vibrator aufweist.

Gemäß der Erfindung sind die Kontaktstifte der Kappe im Boden angeordnet, wobei die zwei Kontaktstifte, die die Kappenkontaktelemente bilden, den Boden durchdringen. Dabei stehen die Kontaktstifte sowohl zur Oberseite als auch zur Unterseite des Bodens vor, wobei einer der Kontaktstifte in einem elektrisch leitenden Kontakt mit der zweiten Elektrode steht.

Weiterhin sieht die Erfindung vor, dass am Boden eine umlaufende Gummidichtung angebracht ist, die den Boden gegenüber der Innenwand der Kappe dichtet, so dass die Kammer in der Spitze der Kappe, in der der Vibrator aufgenommen ist, vor Wasser und Feuchtigkeitseintritt geschützt ist.

Weiterhin ist vorgesehen, dass von der Unterseite des Bodens zwei Laschen abstehen, von denen eine mit einem Schlitz versehen ist. Die Kappe mit dem Vibrator soll nämlich mit Basisgeräten, die sich schon auf dem Markt befinden, kompatibel sein. Diese weisen eine Raste auf, die in einen Schlitz in der Innenwand der Kappe eingreift, um diese am Basiskörper zu halten. Da die Kappe mit dem Vibrator verlängert ist und sich in ihrem unteren Bereich deutlich erweitert, werden die Laschen benötigt, um die Kappe am Aufsteckbereich des Basisgerätes zu fixieren, wobei der Schlitz als Pendant zur Raste am Basiskörper fungiert.

Damit ein Kontakt zur zweiten Elektrode gebildet wird, ist in der Kappe eine Steckerbuchse ausgebildet, deren Innenseite in einem elektrisch leitenden Kontakt mit der zweiten Elektrode steht und die so ausgerichtet ist, dass der eine Kontaktstift in die Steckerbuchse eingeführt ist, wenn der Boden in die Kappe eingesetzt ist.

Der Boden mit den beiden Kontaktstiften braucht daher lediglich in die Kappe eingedrückt zu werden. Dabei wird einer der Kontaktstifte in die Steckerbuchse eingeführt und erhält eine leitende Verbindung zur zweiten Elektrode. Die zur Unterseite des Bodens hervorstehenden Stiftenden gelangen in Kontakt zu zwei Basiskontaktstiften am Basiskörper, mit denen eine schaltbare Verbindung zur Energiequelle im Basiskörper hergestellt wird.

Um die Stromversorgung des elektrischen Antriebs des Vibrators zu erzielen, werden die oberen Enden der Kontaktstifte mit Lötfahnen versehen, an denen die Stromzuleitungskabel zum elektrischen Motor angelötet werden. Die Erfindung sieht daher vor, dass an den Enden der Kontaktstifte die auf der Oberseite des Bodens hervorstehenden Lötfahnen aufgesteckt sind.

Um den Boden noch besser in der Kappe zu fixieren und die durch den Vibrator angeregten Schwingungen des Bodens noch besser auf die Kappe bzw. auf den Bereich der Kappe, die die Elektrode bildet, zu übertragen, ist vorgesehen, dass der Boden von zwei Haltestiften durchdrungen ist, die zur Oberseite vorstehen. In den Kappen befinden sich Haltebuchsen, in die die Haltestifte eingeführt oder eingeschraubt sind, wenn der Boden in die Kappe eingesetzt ist.

Vorzugsweise sind in der Kappe von der Spitze der Kappe ausgehende Säulen angeordnet, wobei die Haltebuchsen in den Säulen ausgebildet sind und ihre Öffnungen in die Stirnseiten der Säulen münden.

Denkbar wäre auch, dass in der Kappe Querstege angeordnet sind, die gegenüberliegende Abschnitte der Kappe miteinander verbinden, und dass die Haltebuchsen in den Querstegen ausgebildet sind.

Die Kappe schließt einen Raum in der Spitze der Kappe ein, in der der Vibrator aufgenommen ist, wobei die Schwingungen des Vibrators über den Halter und den Boden auf die Kappe übertragen werden.

Um den Aufbau des Gerätes einfach zu halten, ist vorgesehen, dass eine vom Nutzer des Gerätes einstellbare Schalteinrichtung im Basiskörper vorhanden ist, die die elektrischen Verbindungen der Energiequelle zu den Elektroden herstellt, und dass auch der vibrator über die Schalteinrichtung mit der elektrischen Energiequelle verbunden ist. Es liegt somit eine gemeinsame Energiequelle für die Elektrode und den Vibrator vor.

Vorzugsweise ist die Schalteinrichtung mit einem elektronischen Speicher versehen, in dem die Parameter von auszuwählenden Betriebszuständen gespeichert sind, die vom Benutzer mittels einer Taste auswählbar sind, wobei einer der Parameter sich auf die Polarität der Elektroden bezieht, und wobei der Vibrator von der Art ist, dass er unabhängig von der Polarität der angelegten Spannung arbeitet.

Damit der Vibrationseffekt möglichst groß ist, sieht die Erfindung weiterhin vor, dass die Kappe an ihrem von dem Basiskörper entfernten Ende zwei seitlich abstehende Zungen aufweist, deren Oberseiten eine durchgehende Fläche bilden, die in Kontakt mit der zu behandelnden Haut bringbar ist, wobei der Vibrator in einem unmittelbaren mechanischen Kontakt mit wenigstens einer der Zungen steht. Vorzugsweise ist die Oberseite der durchgehenden Fläche mit einer Riffelung versehen, so dass die Wirkung auf die Haut verstärkt wird.

Die Kappen, die für den Betrieb des Gerätes zur Verfügung gestellt werden, brauchen nicht alle mit einem vibrator versehen sein. In einigen Anwendungsfällen reicht es nämlich aus, allein eine Strombehandlung durchzuführen.

Damit die Batterien möglichst selten geladen werden müssen, muss dafür gesorgt werden, dass der Stromverbrauch des Gerätes möglichst minimiert wird. Dies ist insbesondere dann der Fall, wenn das Gerät mit einem Vibrator versehen ist, der die zweite Elektrode, die auf die zu behandelnde Hautfläche aufgesetzt wird, in eine rüttelnde Bewegung versetzt. Um bei einem solchen Gerät den Stromverbrauch zu minimieren, sieht die Erfindung vor, dass eine Einrichtung zum Detektieren eines Stromflusses durch die zweite Elektrode, ein Schalter in der Verbindung des Antriebs des Vibrators zu Batterien und eine Steuerung vorgesehen sind, wobei die Steuerung so eingerichtet ist, dass der Schalter nur dann geschlossen ist, wenn durch die zweite Elektrode ein Strom fließt.

Mit anderen Worten, der vibrator wird nur dann eingeschaltet, wenn die zweite Elektrode auf die zu behandelnde Hautfläche aufgesetzt ist und durch sie ein Strom durch die Haut fließt. Dieser Stromfluss wird detektiert und von einer Steuerung genutzt, um den Schalter zu betätigen. Die Einrichtung lässt sich elektronisch realisieren. Dies bedeutet, dass der Schalter als ein Transistor ausgeführt ist.

Weiterhin ist vorgesehen, dass das Gerät wiederaufladbare Batterien, deren Pole beim Betrieb des Gerätes mit den Elektroden in einer elektrischen Verbindung stehen, aufweist. Die Batterien sind in einem Schacht untergebracht, der von einem schalenförmigen Gehäusedeckel überdeckt ist, dessen Außenseite einen Abschnitt der Außenfläche des Gerätegehäuses bildet. Weiterhin ist eine Ladeschaltung vorgesehen. Der Schacht wird von einer entfernbaren Schachtabdeckung abgedeckt, deren Innenseite zu den Batterien im Schacht weist und auf deren Außenseite eine wenigstens 2-polige Steckerbuchse angeordnet ist, die mit der Ladeschaltung in Verbindung steht. Die Schachtabdeckung befindet sich innerhalb des Gehäusedeckels.

Die Steckerbuchse ist vorzugsweise eine Steckerbuchse nach dem USB Standard.

Im Folgenden soll anhand von Ausführungsbeispielen die Erfindung näher erläutert werden. Dazu zeigen:
- Fig. 1 a, b, c: die Ansichten eines erfindungsgemäßen Gerätes, wobei als Kappe eine gerundete Struktur vorgesehen ist,
- Fig.2 a, b, c: verschiedene Kappenformen zur Verwendung mit dem erfindungsgemäßen Gerät,
- Fig. 3: den Querschnitt einer Kappenform, die für den Betrieb mit einem vibrator besonders geeignet ist,
- Fig. 4: einen Motor mit einem Excenter,
- Fig. 5 a, b: eine Anordnung von Kontaktelementen zur Herstellung eines elektrischen Kontaktes zwischen dem Basiskörper und der Kappe,
- Fig. 6: eine Ausführung einer Rastverriegelung,
- Fig. 7: einen Schaltkreis zum Betrieb des Gerätes,
- Fig. 8: eine weitere Ausführung des erfindungsgemäßen Gerätes, die aber nicht Gegenstand des Patents ist,
- Fig. 9: eine alternative Vibratoranordnung,
- Fig. 10: zwei Außenansichten (von vorne und von der Seite) eines erfindungsgemäßen Gerätes zur Hautbehandlung, bestehend aus einem in der Hand zu haltenden Basiskörper und einer Kappe mit einer eine Elektrode bildenden Plattform zum Aufsetzen auf die Haut,
- Fig. 11: eine perspektivische und transparente Darstellung der Kappe, in deren Inneren ein Boden mit einem daran gehaltenen Vibrator zu erkennen ist,
- Fig. 12: eine perspektivische Darstellung der Kappe mit dem Vibrator,
- Fig. 13: einen Längsschnitt durch die Kappe mit dem Vibrator,
- Fig. 14: einen Längsschnitt (senkrecht zum Längsschnitt der Fig. 13) durch die Kappe mit dem vibrator und dem oberen Teil des Basiskörpers,
- Fig. 15: einen Schnitt durch den Batterieschacht des Gerätes und
- Fig. 16: eine Schaltung für die Steuerung eines Vibrationsmotors.

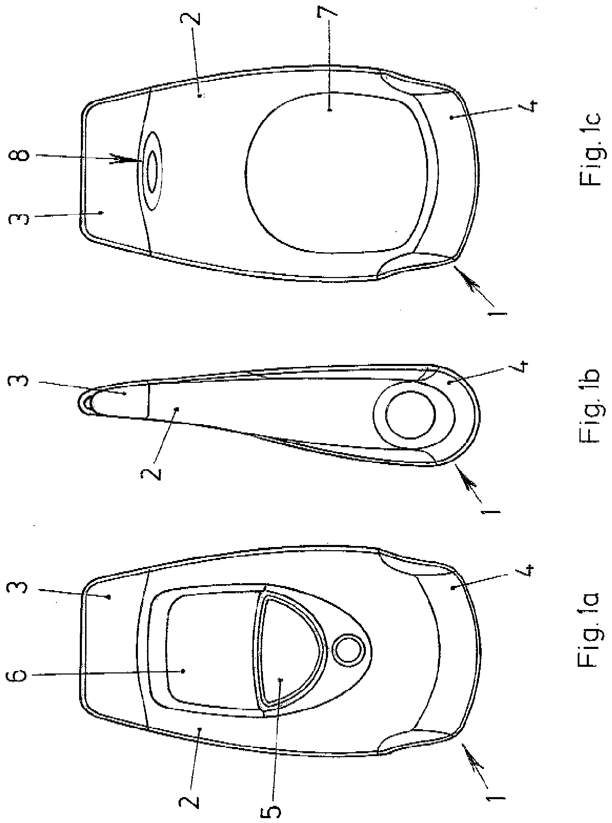

Zunächst wird auf die Fig. 1 Bezug genommen. Wie die drei Ansichten zeigen, ist das erfindungsgemäße Gerät 1 etwa handtellergroß und hat eine rechteckige, flache Form. Es besteht aus einem Basiskörper 2 und einer darauf aufgesteckten Kappe 3. An der unteren kurzen Seite des Basiskörpers 2 befindet sich ein Fach 4 zur Aufnahme von Batterien, die in diesem Ausführungsbeispiel als Energiequelle zum Betrieb des Gerätes 1 fungieren.

An der gegenüberliegenden oberen, kurzen Seite des Basiskörpers 2, die nicht so breit wie die untere Seite ist, ist die auswechselbare Kappe 3 aufgesteckt. Auf der Vorderseite des Basiskörpers 2 ist eine Taste 5 zur Auswahl eines Behandlungsprogramms angeordnet, wobei das ausgewählte Behandlungsprogramm in einem darüber liegenden Display 6 angezeigt wird. Auf der Rückseite befindet sich eine großflächige erste Elektrode 7 und darüber ein Druckknopf 8 zur Auslösung einer Raste, mit der die Kappe 3 am Basiskörper 2 gehalten wird.

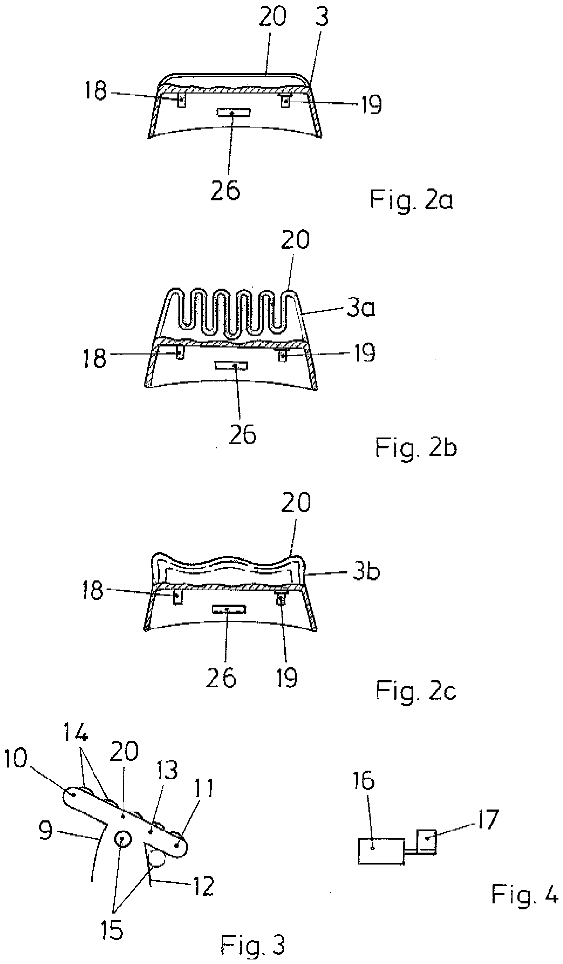

In der Fig. 2 sind im Längsschnitt verschiedene Kappenformen dargestellt. Die Fig. 2a zeigt die schon in der Fig. 1 dargestellte Kappe 3, diese hat eine gerundete obere Abschlusskante.

Die Kappe 3a gemäß der Fig. 2b hat eine kammartige Struktur und eignet sich daher besonders zur Behandlung der Kopfhaut. Die Fig. 2c zeigt eine Kappe 3b mit einer wellenförmig ausgeführten Kante, wodurch der Druck auf die Haut punktuell verstärkt werden kann, so dass beim Hin- und Hergehen der Kappe 3b auf der Haut ein Massageeffekt entsteht.

Gemäß der Fig. 3 ist im Querschnitt eine weitere Kappe 9 dargestellt, die eine Abwandlung der Kappe 3 gemäß Fig. 2a darstellt. An der oberen Kante der Kappe 9 schließen sich zwei Zungen 10, 11 an, die zusammen eine gegenüber einer Basis 12 der Kappe 9 schräg gestellte Plattform 13 bilden. Die Oberseite der Plattform 13 bildet eine durchgehende Fläche, die auf die Haut aufsetzbar ist und die mit Rippen 14 versehen ist.

Diese Form ist besonders geeignet zur Verbindung mit einem Vibrator 15. Dieser ist entweder vorzugsweise im spitzen Winkel zwischen der einen zunge 11 und der Basis 12 der Kappe 9 angebracht (strichpunktiert) oder im Kopf der Basis 12 der Kappe 9 (durchgehende Linie). Denkbar wäre auch eine Unterbringung in einer der Zungen 10, 11.

Der Vibrator 15 besteht im einfachsten Fall gemäß der Fig. 4 aus einem Elektromotor 16, an dessen Welle ein Excenter 17 angebracht ist. Die Ausrichtung der Welle im Bezug zum Gerät ist beliebig. Möglich ist eine Ausrichtung parallel zur oberen Kante der Kappe 9.

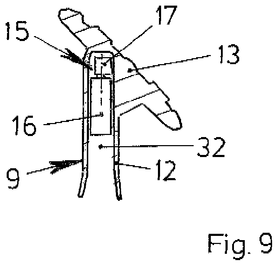

In der Fig. 9 ist eine weitere mögliche Anordnung des Elektromotors 16 des Vibrators 15 gezeigt. Dazu besitzt die Basis 12 der Kappe 9 eine zylindrische Ausformung 32, deren Achse sich in Richtung auf die Plattform 13 erstreckt. In dieser Ausführung ist der Elektromotor 16 koaxial zur Achse der Ausformung 32 angeordnet, wobei seine Welle mit dem Excenter 17 in die Plattform 13 hineinragt. Bei dieser Ausbildung steht die Welle mit dem Excenter 17 senkrecht zur oberen Kante der Kappe 9 und erstreckt sich somit zwischen dem unteren Bereich der Kappe 9, mit der diese auf den Basiskörper 2 aufgesteckt ist, und dem oberen Bereich der Kappe 9, die eine zweite äußere Elektrode 20 bildet.

Wie man den Fig. 2a bis 2c entnehmen kann, befinden sich innerhalb der Kappe 3, 3a, 3b zwei metallische Kontaktstifte 18, 19, wobei der eine Kontaktstift 18 mit der Kappe 3 in elektrischer Verbindung steht und der andere Kontaktstift 19 isoliert gegenüber der Kappe 3, 3a, 3b ist. Die Kappen 3, 3a, 3b bestehen aus einem leitfähigen Material oder besitzen zumindest einen Überzug aus einem leitfähigen Material, das die zweite Elektrode 20 bildet. Der erste Kontaktstift 18 steht somit in Verbindung zu diesem Material, während der zweite Kontaktstift 19 dazu isoliert ist.

Die Kappe 9 gemäß der Fig. 3 ist - was nicht näher gezeigt ist - in gleicher Weise mit Kontaktstiften versehen.

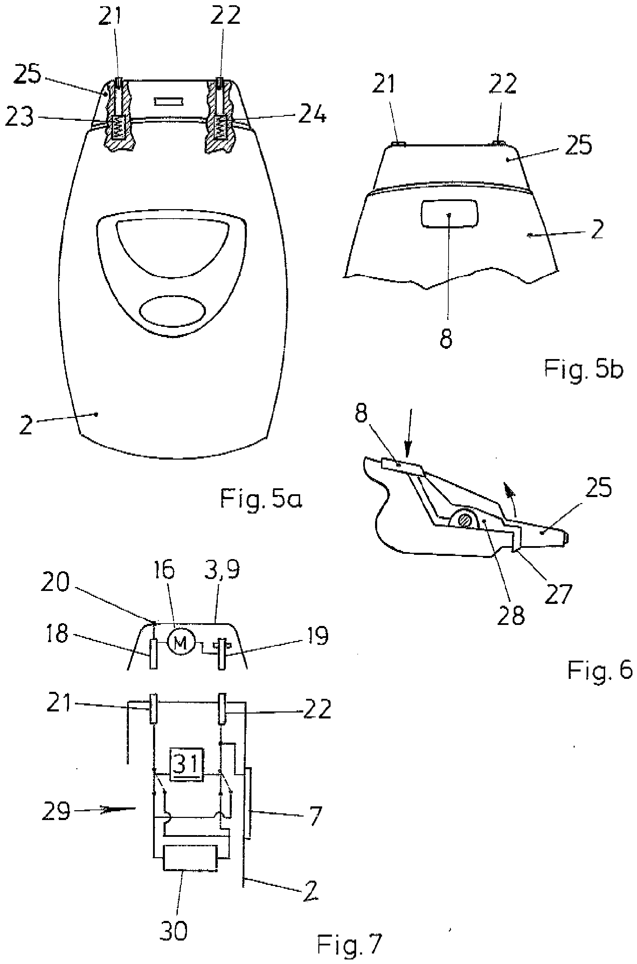

Wie der Fig. 5 zu entnehmen ist, sind an der Oberkante des Basiskörpers 2 zwei federnd gelagerte metallische Gegenstifte 21, 22 angebracht, die fluchtend zu den Kontaktstiften 18, 19 im Basiskörper 2 ausgerichtet sind. Die Gegenstifte 21, 22 werden jeweils von einer in einem Absatz 25 angeordneten Feder 23, 24 gegen einen Anschlag gedrückt, so dass sie ein wenig über die Oberkante des Absatzes 25 hervorragen. Wird die Kappe 3a, 3b, 3c, 9 auf den Absatz 25 des Basiskörpers 2 aufgesteckt, entstehen somit zwei geschlossene elektrische Kontakte.

Wie in Fig. 2 dargestellt, befindet sich in den Kappen 3a, 3b, 3c jeweils ein Schlitz 26 für eine Raste. Entsprechend ist die Kappe 9 gemäß der Fig. 3 ausgebildet. Wie die Fig. 6 zeigt, befindet sich eine solche Raste 27 am Ende eines Hebels 28, der drehbar am Basiskörper 2 gelagert ist und dessen anderes Ende in Verbindung mit dem Druckknopf 8 steht, so dass die Raste 27 aus dem Schlitz 26 herausgezogen wird, wenn der Druckknopf 8 betätigt wird.

Wie den Fig. 5a, 5b zu entnehmen ist, sind die Gegenstifte 21, 22 am Basiskörper federnd gelagert, so dass sie einen Druck auf die Kontaktstifte 18, 19 in der Kappe 3, 9 ausüben, Wird der Druckknopf 8 betätigt, drücken die Gegenstifte 21, 22 die Kappe 3, 3a, 3b, 9 leicht vom Basiskörper 2 weg, so dass sie einfach entfernt werden kann.

In der Fig. 7 ist schematisch ein Schaltplan dargestellt. Der Antrieb des Vibrators, also im Ausführungsbeispiel der Elektromotor 16, ist zwischen den beiden Kontaktstiften 18, 19 in der Kappe 3, 9 geschaltet, während der erste Kontaktstift 18 nur mit der zweiten Elektrode 20 an der Kappe 3, 9 verbunden ist.

Am Basiskörper 2 befinden sich die beiden Gegenstifte 21, 22, die über eine Schalteinrichtung in Form eines Wechselschalters 29 mit einer Spannungsquelle 30 (Batterien) verbunden sind. Der Gegenstift 21, der mit dem isolierten Kontaktstift 18 in der Kappe 3, 9 in Kontakt tritt, hat eine Verbindung zur ersten Elektrode 7 am Basiskörper 2.

Wird nun die Kappe 3, 9 - wie in der Fig. 7 angedeutet - auf den Basiskörper 2 aufgesteckt (eine Codierung verhindert dabei eine Vertauschung der Kontakte), entsteht ein erster Stromkreis, bestehend aus der Spannungsquelle 30 und dem Elektromotor 16, der über die beiden Stiftpaare verläuft, sowie im Betrieb ein zweiter Stromkreis, der über den ersten Kontakt der zweiten Elektrode 20 über den Körper des Nutzers zur ersten Elektrode 7 und zurück zur Spannungsquelle 30 verläuft.

Der Wechselschalter ist in Form einer Transistorschaltung realisiert, die von einer Steuereinrichtung 31 angesteuert wird. Die Steuereinrichtung 31 enthält einen elektronischen Speicher, in dem mehrere Betriebsarten abgespeichert sind. Die Betriebsarten werden durch die Parameter (Dauer der Behandlung, Polarisierung der Elektroden, Zuschaltung des Vibrators) bestimmt. Der Nutzer kann durch Betätigung der Taste 5 eine bestimmte Betriebsart auswählen, die in dem Display 6 angezeigt wird.

Eine Hautbehandlung besteht in der Regel aus einem ersten Schritt, bei dem zunächst eine Reinigungscreme aufgebracht wird und das Gerät in einer ersten Polung betrieben wird, wobei durch den Stromfluss Schadstoffe aus der Haut in die Reinigungscreme übergehen. Hierbei werden Kappen 3 genutzt, die keinen Vibrator enthalten, weil die Creme gerade nicht in die Haut eingearbeitet werden soll.

In einem zweiten Schritt, nachdem die Reinigungscreme entfernt wurde, wird eine Behandlungscreme aufgetragen und das Gerät 1 in einer zweiten Polung betrieben, so dass die Wirkstoffe aus der Behandlungscreme in die Haut eindringen können. Bei dieser Vorgehensweise werden Kappen 9 benutzt, die einen Vibrator 15 aufweisen, so dass neben dem Stromeffekt, der die Wirkstoffe in die Haut transportiert, auch ein Einmassageeffekt vorhanden ist, mit dem der Wirkstoff in die Haut einmassiert wird. Neben der erhöhten Effektivität wird auch ein angenehmes Behandlungsgefühl erzeugt, da eine vibrierende Massage vom Nutzer als angenehm empfunden wird.

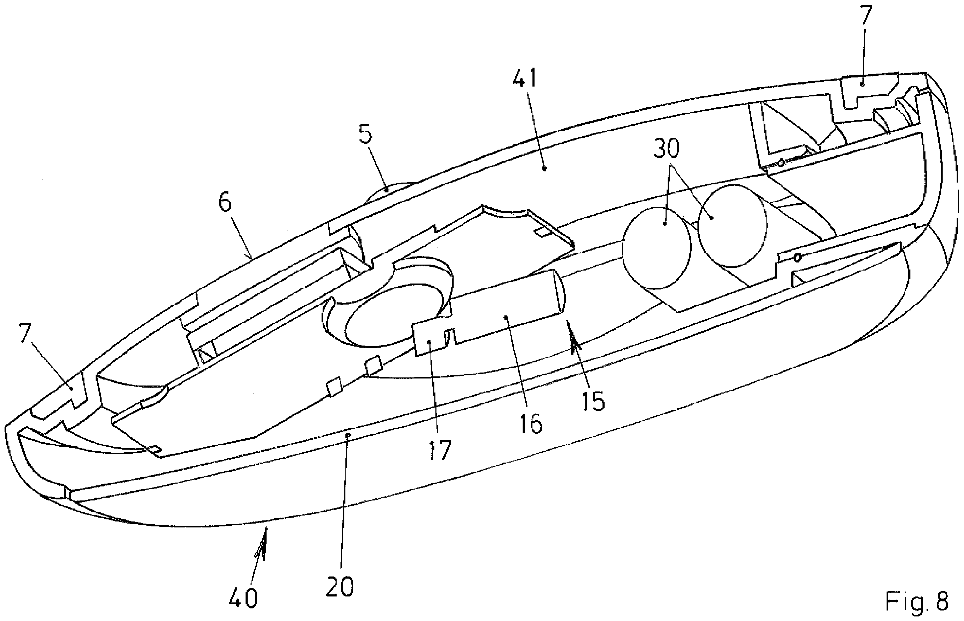

Die Fig. 8 zeigt im Querschnitt ein Gerät 40, das insbesondere zur Zellulitis-Behandlung von großen Hautflächen geeignet ist.

An der Unterseite eines flachen Gehäuses 41 befindet sich eine fast die gesamte Unterseite einnehmende zweite Elektrode 20, die auf die zu behandelnde Hautfläche aufgesetzt wird. Auf der Oberseite befinden sich ein Display 6 und eine Taste 5 zur Auswahl eines Behandlungsprogramms.

Am Außenrand der Oberseite des Gehäuses 41 befindet sich umlaufend eine erste Elektrode 7, die in Kontakt zu der das Gerät 40 haltenden Hand steht. Innerhalb des Gehäuses 41 befinden sich eine Spannungsquelle 30 in Form von Batterien, sowie ein Vibrator 15, der aus einem Elektromotor 16 mit einem Excenter 17 besteht. Der Vibrator 15 ist zentral oberhalb der zweiten Elektrode 20 mittels eines nicht näher dargestellten Sockels an der unteren Wand des Gehäuses 41 angebracht und setzt somit die Wand zusammen mit der zweiten Elektrode 20 in Schwingung, wenn er in Betrieb genommen wird.

Die Verschaltung der Elektroden 7, 20 des Elektromotors 16 und der Spannungsquelle 30 entspricht der, die in Fig. 7 gezeigt ist, wobei allerdings die Stifte 1B, 19; 21, 22 durch durchgehende Verbindungen ersetzt sind. Die Stifte werden nicht benötigt, da das Gerät 40 in dieser Ausführung keine auswechselbaren Kappen 3, 9 mit zweiten Elektroden 20 besitzt.

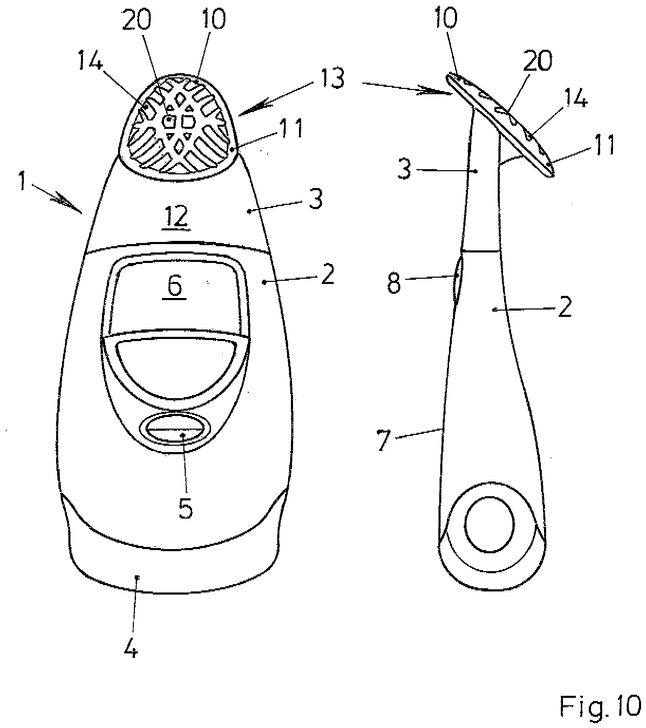

Im Folgenden wird auf die Fig. 10 Bezug genommen. Wie die zwei Ansichten zeigen, ist das erfindungsgemäße Gerät 1 etwa handtellergroß und hat eine rechteckige, flache Form. Es besteht aus einem Basiskörper 2 und einer darauf aufgesteckten Kappe 3. An der unteren kurzen Seite des Basiskörpers 2 befindet sich ein Fach 4 zur Aufnahme von Batterien, die in diesem Ausführungsbeispiel als Energiequelle zum Betrieb des Gerätes 1 fungieren.

An der gegenüberliegenden oberen, kurzen Seite des Basiskörpers 2, die nicht so breit wie die untere Seite ist, ist die auswechselbare Kappe 3 aufgesteckt. Auf der Vorderseite des Basiskörpers 2 ist eine Taste 5 zur Auswahl eines Behandlungsprogramms angeordnet, wobei das ausgewählte Behandlungsprogramm in einem darüber liegenden Display 6 angezeigt wird. Auf der Rückseite befindet sich eine großflächige erste Elektrode 7 und darüber ein Druckknopf 8 zur Auslösung einer Raste, mit der die Kappe 3 am Basiskörper 2 gehalten wird.

An der oberen Kante der Kappe 3 schließen sich zwei Zungen 10, 11 an, die zusammen eine gegenüber der Basis 12 der Kappe 3 schräg gestellte Plattform 13 bilden. Die Oberseite der Plattform 13 bildet eine durchgehende Fläche, die auf die Haut aufsetzbar ist und die mit Rippen 14 versehen ist.

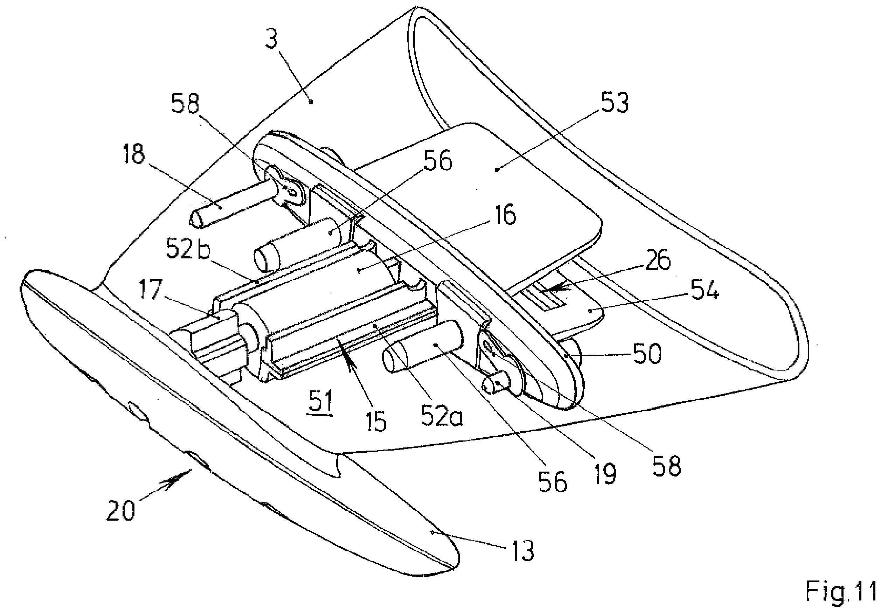

Der Vibrator 15 besteht im einfachsten Fall gemäß den Fig. 11, 3, 4 und 5 aus einem Elektromotor 16, an dessen Welle ein Excenter 17 angebracht ist.

Innerhalb der Basis 12 der Kappe 3 befindet sich eine zylindrische Ausformung 32, deren Achse sich in Richtung auf die Plattform 13 erstreckt. In dieser Ausführung ist der Elektromotor 16 koaxial zur Achse der Ausformung 32 angeordnet, wobei seine Welle mit dem Excenter 17 in die Plattform 13 hineinragt. Bei dieser Ausbildung steht die Welle mit dem Excenter 17 senkrecht zur oberen Kante der Kappe 3 und erstreckt sich somit zwischen dem unteren Bereich der Kappe 3, mit der diese auf den Basiskörper 2 aufgesteckt ist, und dem oberen Bereich der Kappe 3, die die zweite äußere Elektrode 20 bildet.

Wie man den Fig. 11, 12, 13, 14 weiterhin entnehmen kann, befinden sich innerhalb der Kappe 3 zwei metallische Kontaktstifte 18, 19, wobei der eine Kontaktstift 18 mit der Kappe 3 in elektrischer Verbindung steht und der andere Kontaktstift 19 isoliert gegenüber der Kappe 3 ist. Die Kappe 3 mit der Plattform 13 besteht aus einem leitfähigen Material oder besitzt zumindest einen Überzug aus einem leitfähigen Material, so dass die Plattform 13 die zweite Elektrode 20 bildet.

Die Fig. 11 zeigt eine perspektivische Darstellung der Kappe 3, die transparent dargestellt ist, so dass ein Boden 50, der in die Kappe eingesetzt ist, zu erkennen ist. Der Boden 50 befindet sich in etwa auf halber Höhe in der Kappe 3, wobei seine Umfangskontur dem Querschnitt der Kappe 3 an dieser Stelle entspricht, so dass der Boden 50 eine Kammer 51 in der Spitze der Kappe unterhalb der Plattform 13 einschließt. Auf der der Plattform 13 zugewandten Oberseite des Bodens 50 befinden sich zwei vom Boden 50 senkrecht abstehende Arme 52a, 52b, die die Ausformung 32 für den Elektromotor 16 bilden. Zwischen den Armen 52a, 52b befindet sich der Elektromotor 16, dessen Welle mit dem Excenter 17 in Richtung auf die Plattform ausgerichtet ist. An der Unterseite des Bodens 50 befinden sich zwei Laschen 53 und 54, die parallel zueinander ausgerichtet sind und die einen hier nicht dargestellten Aufsteckbereich am Basiskörper beidseitig umgreifen. In der einen Lasche 54 befindet sich ein Schlitz 26, in dem eine hier ebenfalls nicht dargestellte Raste am Basiskörper eingreift, um die Kappe 3 am Basiskörper zu halten.

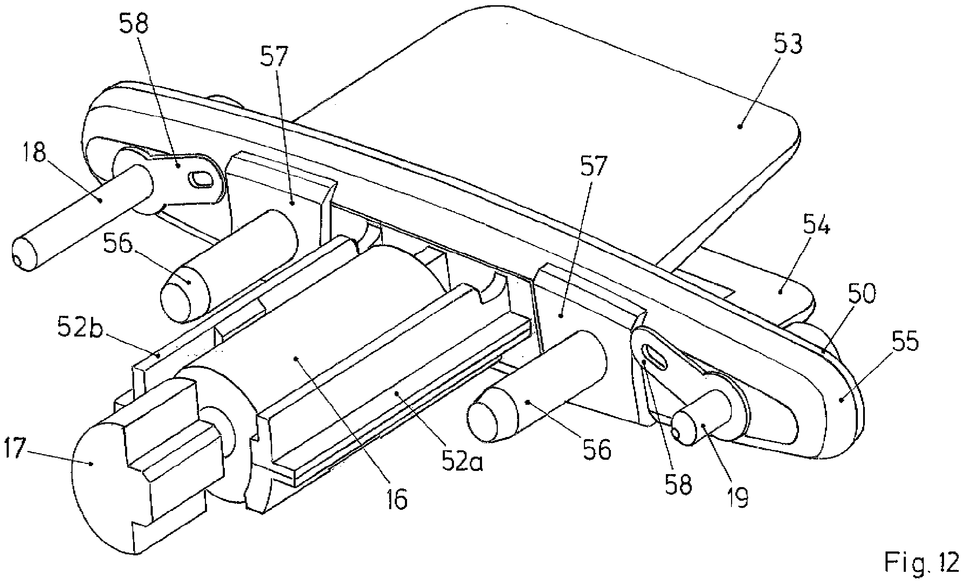

Der Boden 50 ist in der Fig. 12 nochmals vergrößert dargestellt. Er weist eine umlaufende Gummidichtung 55 auf, die sich an die Innenwand der Kappe 3 anschmiegt. Die beiden Arme 52a und 52b befinden sich im mittleren Teil des Bodens 50 und sind an ihren aneinander zugewandten Seiten zumindest teilweise zylindrisch gekrümmt ausgeführt, wobei die Einhüllende der Krümmungen einen Zylinder bildet, der den zylindrischen Elektromotor 16 aufnimmt.

Links und rechts der beiden Arme 52a, 52b befindet sich je ein Haltestift 56, der senkrecht vom Boden 50 absteht und der jeweils von einer Dichtungsplatte 57 umgeben ist. Die beiden Dichtungsplatten 57 bilden eine Einheit mit der Gummidichtung 55.

Weiter außen am Boden 50 befindet sich der erste Kontaktstift 18 und der zweite Kontaktstift 19, wobei der erste Kontaktstift 18 länger ausgeführt ist als der zweite Kontaktstift 19. Auf den Kontaktstiften 18 und 19 ist je eine Lötfahne 58 aufgesteckt.

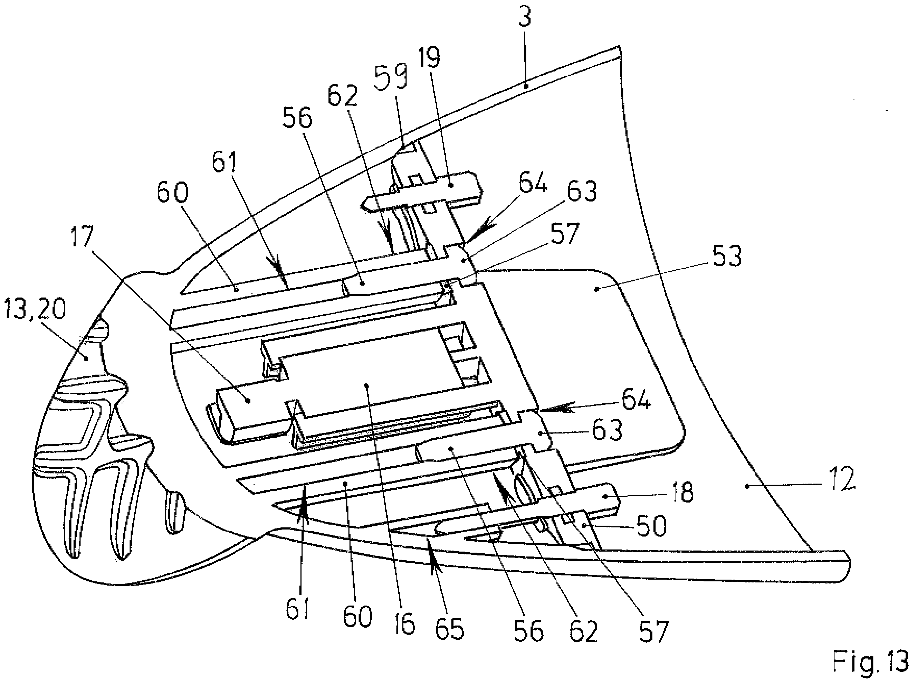

Die Verbindung des Bodens 50 mit der Kappe 3 lässt sich gut der Querschnittsdarstellung der Fig. 13 entnehmen. Die Basis 12 der Kappe weist eine sich konisch erweiternde Form auf, so dass der Boden 50 von unten in die Kappe 3 geschoben werden kann, bis er gegen eine Stufe 59 stößt. Von der Spitze der Kappe 3 gehen zwei Säulen 60 aus, die je einen zylindrischen Kanal 61 aufweisen. In diese Kanäle 61 werden die Haltestifte 56 eingesteckt oder eingeschraubt, wobei sich die Stirnseiten der Säulen 60 dichtend an die Dichtungsplatten 57 anlegen. Die Haltestifte 56 weisen Köpfe 63 auf, die in Vertiefungen 64 an der Unterseite des Bodens 50 liegen. Die Haltestifte 56 werden somit klemmend in den Kanälen 61 gehalten bzw. in diese eingeschraubt, die Haltebuchsen 62 für die Haltestifte 56 bilden, so dass der Boden 50 in der Kappe 3 fixiert ist.

Die Haltestifte 56 haben aber nicht nur die Aufgabe, den Boden 50 in der Kappe 3 zu halten, sondern stellen auch eine schwingungsübertragende Verbindung zur Spitze der Kappe 3 her, wo sich die Plattform 13 befindet. Die durch den sich drehenden Excenter 17 angeregten Schwingungen werden somit unmittelbar in die Plattform 13 übertragen.

Die Kontaktstifte 18, 19 stecken in Durchgangsbohrungen im Boden 50, wobei die unteren Enden zur Unterseite des Bodens 50 hervorstehen und dort in Kontakt treten mit hier nicht dargestellten Gegenkontaktstiften am Basiskörper.

Wie schon erläutert, sind die Enden der Kontaktstifte 18, 19 mit Lötfahnen 58 versehen. Das Ende des zweiten Kontaktstiftes 19 endet frei in der Kammer 51, während der erste Kontaktstift 18 in eine Steckerbuchse 65 an der Innenseite der Kappe 3 eindringt und somit einen elektrisch leitenden Kontakt mit der Kappe 3 herstellt. Diese ist wieder dadurch gegeben, dass die Kappe 3 selbst insgesamt aus einem Metall hergestellt ist oder aber aus einem Kunststoff, der mit einem metallischen Überzug versehen ist, der sich in die Steckerbuchse 65 hinein erstreckt.

Die gezeigte Kappe 3 für ein Hautbehandlungsgerät besteht somit aus dem Basiskörper 2 und der Kappe 3, in der ein vibrator 15, der an einem Boden 50 befestigt ist, eingesetzt wird. Der Boden 50 wird in die Kappe 3 eingesteckt und darin von Haltestiften 56 gehalten. Kontaktstifte 18, 19 stellen einerseits eine elektrische Verbindung zu der zweiten Elektrode 20 an der Kappe 3 und eine Stromversorgung für den Elektromotor 16 des Vibrators her. Die beiden Laschen 53, 54 ermöglichen es, dass auf die Kappe auch weiterhin auch schon bekannte Basiskörper aufgesteckt werden können und mit dieser kompatibel sind.

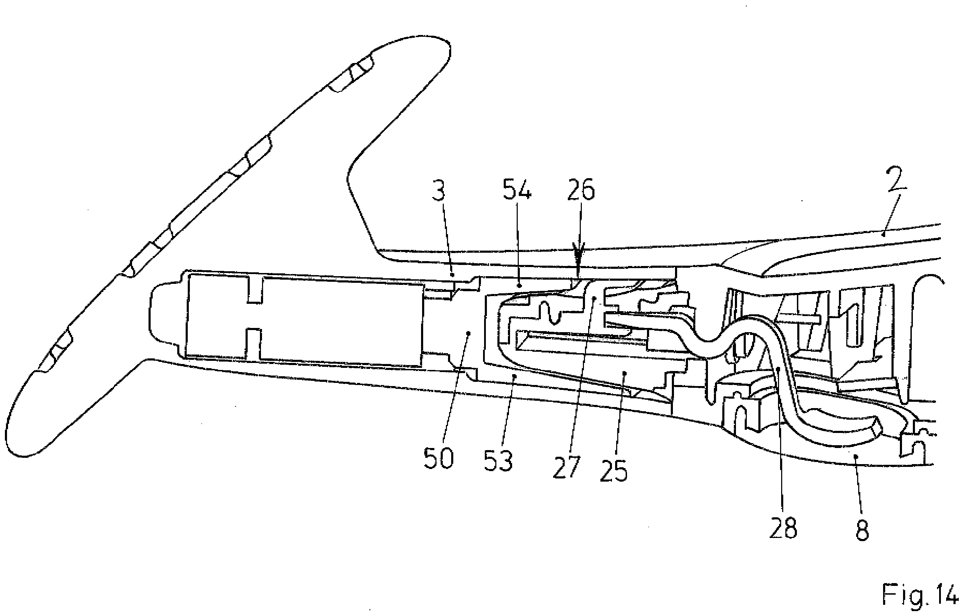

Die Fig. 14 zeigt einen Längsschnitt durch eine auf einen Basiskörper 2 aufgesetzte Kappe 3. Ein von einem Absatz 25 gebildeter Aufnahmebereich des Basiskörpers 2 erstreckt sich zwischen den beiden Laschen 53 und 54, wodurch sich die beiden Laschen 53, 54 keilförmig an die Innenwand der Kappe 3 anlegen. In dem Aufnahmebereich findet sich eine Raste 27, die in den Schlitz 26 der einen Lasche 54 hineinragt. Die Raste wird über einen Hebel 28 betätigt, der vom Druckknopf 8 betätigt wird.

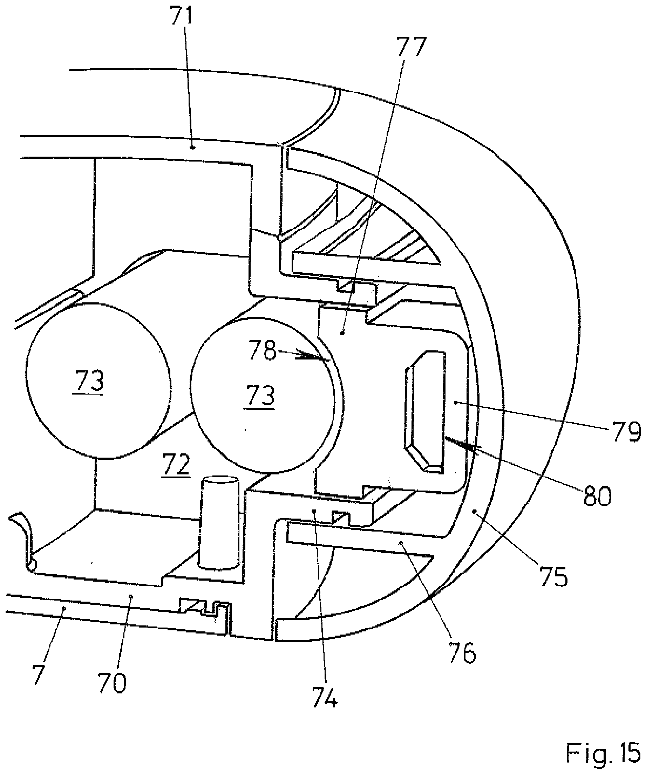

Wie der Fig. 15 entnommen werden kann, besteht das Gehäuse des Gerätes 1 aus zwei Halbschalen 70, 71, die zu einem geschlossenen Gehäuse zusammengesetzt werden, wobei der Bodenbereich des Gehäuses im Wesentlichen von einem Randabschnitt einer der Halbschalen 70 gebildet wird. In diesem Rand ist der Zugang zu einem Schacht 72 untergebracht, in dem wiederaufladbare Batterien 73 eingesetzt werden. Der Schacht 72 wird von einem umlaufenden Steg 74 auf dem Randabschnitt der Halbschale 70 gebildet. In dem Schacht 72 liegen die Batterien 73 antiparallel nebeneinander.

Ein halbschalenförmiger Gehäusedeckel 75 deckt den Schacht 72 ab. Dazu besitzt er an seiner Innenseite ebenfalls einen umlaufenden Gegensteg 76, der auf den Steg 74 an der Halbschale 70 aufgesteckt wird. Die Außenkontur des Gehäusedeckels 75 ist dabei so geformt, dass sich ein glatter Übergang zu den Außenkonturen der Halbschalen 70, 71 bildet.

In dem Schacht 72 selbst ist weiterhin eine Schachtabdeckung 77 vorgesehen, die auf den Batterien 73 aufliegt und dazu an ihrer Innenseite eine rinnenförmige Vertiefung 78 aufweist und die an ihrer Außenseite eine Ausformung besitzt, die ein Steckergehäuse 79 bildet. Das Steckergehäuse 79 liegt an der Innenseite des Gehäusedeckels 75 an, so dass auf diese Weise eine Haltekraft auf die Batterien 73 ausgeübt wird, wenn der Gehäusedeckel 75 mit seinem Gegensteg 76 auf den umlaufenden Steg 74 aufgesteckt ist.

Das Steckergehäuse 79 ist hohl ausgeführt und ist zur Unterseite der Schachtabdeckung 77 hin offen. An einer Wand des Steckergehäuses 79 befindet sich eine Gehäuseöffnung 80. In das Steckergehäuse 79 ist eine hier nicht gezeigte Steckerplatine mit einer USB-Steckerbuchse derart eingesetzt, dass die Steckeröffnung sich vor der Gehäuseöffnung 80 befindet.

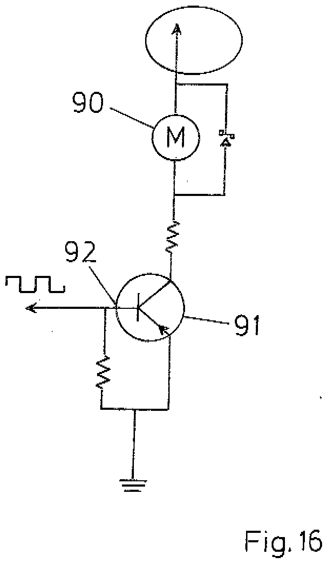

Wie man der Fig. 16 entnehmen kann, ist der Antrieb 90 eines Vibrators in Reihe geschaltet mit der Kollektor/Emitter/Strecke eines Transistors 91. An der Basis 92 des Transistors 91 wird eine Steuerspannung angelegt. Liegt diese vor, schaltet der Transistor durch, so dass der Motor Strom durchflossen ist. Liegt keine Spannung an der Basis 92 an, sperrt der Transistor 91, so dass der Antrieb 90 abgeschaltet wird.

Die Schaltspannung an der Basis wird von einer Steuerschaltung geliefert, die mittels eines Detektors feststellt, ob ein Strom zur zweiten Elektrode fließt.

### Bezugszeichenliste

- 1: Gerät
- 2: Basiskörper
- 3: Kappe
- 3a: Kappe
- 3b: Kappe
- 4: Fach
- 5: Taste
- 6: Display
- 7: erste Elektrode
- 8: Druckknopf
- 9: Kappe
- 10: Zunge

- 11: Zunge
- 12: Basis einer Kappe
- 13: Plattform
- 14: Rippen
- 15: Vibrator
- 16: Elektromotor
- 17: Excenter
- 18: Kontaktstift
- 19: Kontaktstift
- 20: zweite Elektrode

- 21: Gegenstift
- 22: Gegenstift
- 23: Feder
- 24: Feder
- 25: Absatz
- 26: Schlitz
- 27: Raste
- 28: Hebel
- 29: Wechselschalter
- 30: Spannungsquelle
- 31: Steuereinrichtung
- 32: Ausformung
- 40: Gerät
- 41: Gehäuse
- 50: Boden

- 51: Kammer
- 52a: Arm
- 52b: Arm
- 53: Lasche
- 54: Lasche
- 55: Gummidichtung
- 56: Haltestift
- 57: Dichtungsplatte
- 58: Lötfahne
- 59: Stufe
- 60: Säule

- 61: Kanal
- 62: Haltebuchse
- 63: Kopf
- 64: Vertiefung
- 65: Steckerbuchse
- 70: Halbschale
- 71: Halbschale
- 72: Schacht
- 73: Batterien
- 74: Steg
- 75: Gehäusedeckel
- 76: Gegensteg
- 77: Schachtabdeckung
- 78: Vertiefung
- 79: Steckergehäuse
- 80: Gehäuseöffnung

- 90: Antrieb
- 91: Transistor
- 92: Basis

## Patentansprüche

1. In einer Hand haltbares Gerät zur elektrisch unterstützten Hautbehandlung, aufweisend:
Einen Basiskörper (2), der ausgebildet ist, um von einer Hand ergriffen zu werden,
eine erste äußere Elektrode (7), die in Kontakt zur Hand steht, wenn das Gerät vom Nutzer zum Gebrauch in der Hand gehalten wird, wobei die erste äußere Elektrode (7) an dem Basiskörper (2) vorhanden ist,
eine zweite äußere Elektrode (20), die auf den zu behandelnden Hautbereich aufsetzbar ist, und
eine elektrische Energiequelle, deren Pole beim Betrieb des Gerätes mit den Elektroden (7, 20) in einer elektrischen Verbindung stehen, wobei die elektrische Energiequelle in dem Basiskörper (2) angeordnet ist,
eine Kappe (3, 9) zum lösbaren Aufstecken auf den Basiskörper (2), die die zweite äußere Elektrode (20) aufweist, die auf den zu behandelnden Hautbereich aufsetzbar ist,
und einen elektrisch betriebenen Vibrator (15),
**dadurch gekennzeichnet, dass**
der elektrisch betriebene Vibrator (15) in oder an der Kappe (3, 9) angebracht ist, und derart ausgebildet ist, dass der Bereich des Gerätes besonders stark vibriert, der sich an dem zu behandelnden Hautbereich befindet, so dass beim Betrieb des Gerätes ein verstärkter Massageeffekt auf der zu behandelnden Haut entsteht, wodurch die Hautporen geweitet werden,
und dass Kontaktmittel an der Kappe und an dem Basiskörper, die zur Herstellung einer elektrischen Verbindung zwischen der zweiten Elektrode und der elektrischen Energiequelle dienen, so ausgebildet und angeordnet sind, dass sie in Kontakt gebracht werden, wenn die Kappe auf den Basiskörper aufgesteckt wird, und dass der Vibrator (15) mit der elektrischen Energiequelle im Basiskörper (2) in einer schaltbaren elektrisch leitenden Verbindung steht.

2. Gerät nach Anspruch 1, **dadurch gekennzeichnet, dass** zwischen dem Basiskörper (2) und der Kappe (3, 9) ein erster und ein zweiter elektrischer Kontakt vorhanden sind, die jeweils aus einem Basiskontaktelement und einem Kappenkontaktelement bestehen, wobei die Basiskontaktelemente im Basiskörper (2) und die Kappenkontaktelemente in der Kappe (3) derart angeordnet sind, dass die Kontakte geschlossen sind, wenn die Kappe (3) auf den Basiskörper (2) aufgesetzt ist, wobei die elektrische Verbindung zwischen der Energiequelle und der zweiten Elektrode (20) über den ersten Kontakt hergestellt ist und wobei der Stromkreis zur Energieversorgung des Vibrators (15) über den ersten und den zweiten Kontakt verläuft .

3. Gerät nach Anspruch 2, **dadurch gekennzeichnet, dass** die Kappe (3) als Ganzes oder eine äußere Schicht der Kappe (3) aus einem elektrisch leitfähigen Material besteht, dass das Kappenkontaktelement des ersten elektrischen Kontaktes mit dem Material in elektrisch leitfähiger Weise in Verbindung steht und dass das Kappenkontaktelement des zweiten elektrischen Kontaktes gegenüber dem leitfähigen Material elektrisch isoliert ist.

4. Gerät nach einem der vorhergehenden Ansprüche, **dadurch gekennzeichnet, dass** der Vibrator (15) aus einem elektrischen Motor (16) besteht, an dessen Welle ein Excenter (17) befestigt ist, und dass die Kappe an ihrem einen Ende, dem unteren Ende, einen Aufsteckbereich hat, der auf den Basiskörper aufsteckbar ist, und an ihrem entgegengesetzten Ende, dem oberen Ende, die zweite äußere Elektrode bildet, und dass die Achse des elektrischen Motors (16) und die Welle des elektrischen Motors (16) längs zu einer gedachten Linie zwischen dem Aufsteckbereich und der zweiten äußeren Elektrode angeordnet sind.

5. Gerät nach einem der Ansprüche 1 bis 3, **dadurch gekennzeichnet, dass** der Vibrator (15) aus einem elektrischen Motor (16) besteht, an dessen Welle ein Excenter (17) befestigt ist, und dass die Kappe an ihrem einen Ende, dem unteren Ende, einen Aufsteckbereich hat, der auf den Basiskörper aufsteckbar ist, und an ihrem entgegengesetzten Ende, dem oberen Ende, die zweite äußere Elektrode bildet, und dass die Achse des elektrischen Motors (16) und die Welle des elektrischen Motors (16) quer zu einer gedachten Linie zwischen dem Aufsteckbereich und der zweiten äußeren Elektrode angeordnet sind.

6. Gerät nach einem der vorhergehenden Ansprüche, **dadurch gekennzeichnet, dass** in der Kappe (3) ein Boden (50), der eine Kammer (51) in der Spitze der Kappe einschließt, eingesetzt ist, der an seiner der Kammer (51) zugewandten Oberseite einen Halter für den Vibrator (15) aufweist.

7. Gerät nach Anspruch 6, **dadurch gekennzeichnet, dass** von der Kammer (51) abgewandten Unterseite des Bodens (50) zwei Laschen (53, 54) abstehen, von denen eine mit einem Schlitz (26) versehen ist.

8. Gerät nach Anspruch 6 oder 7, **dadurch gekennzeichnet, dass** der Boden (50) von zwei Kontaktstiften (18, 19), die die Kappenkontaktelemente bilden, durchdrungen ist, die sowohl zur Oberseite als auch zur Unterseite des Bodens (50) vorstehen, wobei einer der Kontaktstifte (18) in einem elektrisch leitenden Kontakt mit der zweiten Elektrode (20) steht.

9. Gerät nach Anspruch 8, **dadurch gekennzeichnet, dass** in der Kappe (3) eine Steckerbuchse (65) ausgebildet ist, deren Innenseite in einem elektrisch leitenden Kontakt mit der zweiten Elektrode (20) steht und die so ausgerichtet ist, dass der eine Kontaktstift (18) in die Steckerbuchse (65) eingeführt ist, wenn der Boden (50) in die Kappe (3) eingesetzt ist.

10. Gerät nach einem der Ansprüche 6 bis 9, **dadurch gekennzeichnet, dass** der Boden von zwei Haltestiften (56) durchdrungen ist, die zur Oberseite vorstehen, wobei sich in der Kappe (3) Haltebuchsen (62) befinden, in die die Haltestifte (56) eingeführt oder eingeschraubt sind, wenn der Boden (50) in die Kappe (3) eingesetzt ist, und, dass in der Kappe (3) von der Spitze der Kappe ausgehende Säulen (60) angeordnet sind, wobei die Haltebuchsen (62) in den Säulen (60) ausgebildet sind und ihre Öffnungen in die Stirnseiten der Säulen (60) münden.

11. Gerät nach einem der vorhergehenden Ansprüche, **dadurch gekennzeichnet, dass** eine vom Nutzer des Gerätes einstellbare Schalteinrichtung im Basiskörper (2) vorhanden ist, die die elektrischen Verbindungen der Energiequelle zu den Elektroden (7, 20) herstellt, und dass auch der Vibrator (15) über die Schalteinrichtung mit der elektrischen Energiequelle verbunden ist, und dass die Schalteinrichtung mit einem elektronischen Speicher versehen ist, in dem die Parameter von auszuwählenden Betriebszuständen gespeichert sind, die vom Benutzer mittels einer Taste (5) auswählbar sind, wobei einer der Parameter sich auf die Polarität der Elektroden bezieht, und dass der Vibrator (15) von der Art ist, dass er unabhängig von der Polarität der angelegten Spannung arbeitet.

12. Gerät nach einem der vorhergehenden Ansprüche, **dadurch gekennzeichnet, dass** die Kappe (3, 9) an ihrem von dem Basiskörper (2) entfernten Ende zwei seitlich abstehende Zungen (10, 11) aufweist, die eine durchgehende Fläche bilden, die in Kontakt mit der zu behandelnden Haut bringbar ist.

13. Gerät nach einem der vorhergehenden Ansprüche, **dadurch gekennzeichnet, dass** eine Einrichtung zum Detektieren eines Stromflusses durch die zweite Elektrode, ein Schalter in der Verbindung des Antriebs des Vibrators zu Batterien und eine Steuerung vorgesehen sind, wobei die Steuerung so eingerichtet ist, dass der Schalter nur dann geschlossen ist, wenn durch die zweite Elektrode ein Strom fließt.

14. Gerät nach einem der vorhergehenden Ansprüche, **dadurch gekennzeichnet, dass** es wiederaufladbare Batterien aufweist, deren Pole beim Betrieb des Gerätes mit den Elektroden in einer elektrischen Verbindung stehen,
und einen Schacht (72) zur Aufnahme der Batterien besitzt, der von einem schalenförmigen Gehäusedeckel (75) überdeckt ist, dessen dem Schacht (72) abgewandte Außenseite einen Abschnitt der Außenfläche der Außenseite des Gerätegehäuses bildet,
wobei eine Ladeschaltung vorgesehen ist, und
der Schacht (72) von einer entfernbaren Schachtabdeckung (77) abgedeckt ist, deren Innenseite zu den Batterien (73) im Schacht weist und auf deren gegenüberliegende Außenseite eine wenigstens 2-polige Steckerbuchse angeordnet ist, und
sich die Schachtabdeckung (77) innerhalb des Gehäusedeckels (75) befindet.

## Claims

1. A hand-held device for electrically powered skin treatment, comprising;
A base (2) which is designed to be grasped by a hand,
a first outer electrode (7) which is in contact with the hand when the device is held in the user's hand for use, said first outer electrode (7) being provided on the base (2),
a second outer electrode (20) which can be placed on the area of skin to be treated,
an electrical energy source, the poles of which are electrically connected to the electrodes (7, 20) during operation of the device, said electrical energy source being arranged in the base (2),
a cap (3, 9) to be fitted detachably on the base (2), said cap comprising the second outer electrode (20), which can be placed on the area of skin to be treated, and
an electrically operated vibrator (15)
**characterised in that**
the electrically operated vibrator (15) is fixed in or on the cap (3, 9) and is adapted to strongly vibrate the part of the device located on the area of skin to be treated so that, during operation, the device produces an intensified massaging effect on the skin to be treated, wherein the skin pores are widened,
and that contact means on the cap and on the base, which serve to establish an electrical connection between the second electrode and the electrical energy source, are designed and arranged in such a manner that they are brought into contact when the cap is placed on the base, and **in that** the vibrator (15) is in switchable electrically conducting contact with the electrical energy source in the base (2).

2. The device according to claim 1, **characterised in that** a first and a second electrical contact are provided between the base (2) and the cap (3, 9), both electrical contacts comprising a base contact element and a cap contact element, the base contact element being arranged in the base (2) and the cap contact element being arranged in the cap (3) in such a way that the contacts are closed when the cap (3) is placed on the base (2), the electrical connection between the energy source and the second electrode (20) being produced by the first contact, and the electric circuit, which supplies power to the vibrator (15), running through the first and second contacts.

3. The device according to claim 2, **characterised in that** the cap (3) consists entirely of an electrically conductive material, or an outer layer of the cap (3) consists of an electrically conductive material, **in that** the cap contact element of the first electrical contact is connected to the material in an electrically conductive manner, and that the cap contact element of the second electrical contact is electrically insulated with respect to the conductive material.

4. The device according to any one of the preceding claims, **characterised in that** the vibrator (15) comprises an electric motor (16), a cam (17) being fastened to the shaft of said electric motor, and that, on its one end, being the lower end, the cap has a plug-on region which can be plugged onto the base, and on its opposite end, being the upper end, it forms the second outer electrode, and **in that** the axle and the shaft of the electric motor (16) extend essentially parallel to a virtual line between the plug-on region and the second outer electrode.

5. The device according to claims 1 to 3, **characterised in that** the vibrator (15) comprises an electric motor (16), a cam (17) being fastened to the shaft of said electric motor, and **in that**, on its one end, being the lower end, the cap has a plug-on region which can be plugged onto the base, and on its opposite end, being the upper end, it forms the second outer electrode, and **in that** the axle and the shaft of the electric motor (16) extend essentially transverse to a virtual line between the plug-on region and the second outer electrode.

6. The device according to any one of the preceding claims, **characterised in that** in the cap (3) a bottom (50), which encloses a chamber (51) in the peak of the cap, is inserted which on its upper side facing the chamber (51) comprises a retainer for the vibrator (15).

7. The device according to claim 6, **characterised in that** two tabs (53, 54) project from the underside of the bottom (50) facing away from the chamber (51), with one of these tabs comprising a slot (26).

8. The device according to claim 6 or 7, **characterised in that** two contact pins (18, 19) that form the cap contact elements penetrate the bottom (50), wherein the contact pins project towards the upper side as well as towards the underside of the bottom (50), wherein one of the contact pins (18) is in electrically conductive contact with the second electrode (20).

9. The device according to claim 8, **characterised in that** in the cap (3) a connector socket (65) is formed, the inside of which is in electrically conductive contact with the second electrode (20), wherein the connector socket is aligned in such a manner that the said contact pin (18) is inserted in the connector socket (65) when the bottom (50) is inserted in the cap (3).

10. The device according to any one of claims 6 to 9,
**characterised in that** the bottom is penetrated by two retaining pins (56) that project towards the upper side, wherein in the cap (3) there are retaining bushes (62), into which the retaining pins (56) are inserted or screwed when the bottom (50) is inserted in the cap (3), and **in that** pillars (60) that emanate from the peak of the cap are arranged in the cap (3), wherein the retaining bushes (62) are formed in the pillars (60) and their openings lead into the end faces of the pillars (60).

11. The device according to any one of the preceding claims, **characterised in that** a switching device, which can be adjusted by the user of the device, is provided in the base (2) and produces the electrical connections between the energy source and the electrodes (7, 20), and **in that** the vibrator (15) is also connected to the electrical energy source by the switching device, and **in that** the switching device is provided with an electronic memory, in which the parameters of operating states to be selected are stored and can be selected by the user by means of a button (5), one of the parameters relating to the polarity of the electrodes, and **in that** the vibrator (15) is of the kind that operates independently of the polarity of the applied voltage.

12. The device according to any one of the preceding claims, **characterised in that** the cap (3, 9) has two laterally protruding tongues (10, 11) on its end distanced from the base (2), the tongues forming a continuous surface which can be brought into contact with the skin to be treated.

13. The device according to any one of the preceding claims, **characterised in that** a device for detecting a current flow through the second electrode, a switch in the connection of the drive of the vibrator, and a control device are provided, wherein the control device is designed in such a manner that the switch is only closed when a current flows through the second electrode.

14. The device according to any one of the preceding claims, **characterised in that** it comprises rechargeable batteries, whose poles during operation of the device are in electrical connection with the electrodes,
and a compartment (72) for accommodating the batteries, wherein the compartment is covered by a shell-like housing cover (75) whose outside facing away from the compartment (72) forms a section of the exterior area of the device housing,
wherein a charging circuit is provided, and
the compartment (72) is covered by a removable compartment cover (77) whose inner side is directed to the batteries (73) in the compartment and on whose outer side a connector socket with at least two poles is arranged, and
the compartment cover (77) is arranged within the housing cover (75).

## Revendications

1. Appareil, pouvant être tenu dans une main, pour le traitement dermatologique à assistance électrique, présentant:
un corps de base (2) réalisé pour être saisi par une main,
une première électrode extérieure (7) en contact avec la main lorsque l'appareil est tenu dans la main pour l'utilisation par l'utilisateur, dans lequel la première électrode extérieure (7) est présente sur le corps de base (2),
une deuxième électrode extérieure (20) pouvant être posée sur la zone de peau à traiter, et
une source d'énergie électrique dont les pôles, lors du fonctionnement de l'appareil, sont en connexion électrique avec les électrodes (7, 20), dans lequel la source d'énergie électrique est disposée dans le corps de base (2),
un capuchon (3, 9), pour la fixation amovible sur le corps de base (2), lequel présente la deuxième électrode extérieure (20) pouvant être posée sur la zone de peau à traiter,
et un vibreur (15) fonctionnant électriquement,
**caractérisé en ce**
**que** le vibreur fonctionnant électriquement (15) est monté dans ou sur le capuchon (3, 9) et est réalisé de telle sorte que la zone de l'appareil qui se trouve au niveau de la zone de peau à traiter vibre de manière particulièrement forte, de sorte que lors du fonctionnement de l'appareil, un effet de massage renforcé est produit sur la peau à traiter, moyennant quoi les pores de la peau sont élargis,
et **que** des moyens de contact sur le capuchon et sur le corps de base, lesquels servent à établir une connexion électrique entre la deuxième électrode et la source d'énergie électrique, sont réalisés et disposés de telle sorte qu'ils sont mis en contact lorsque le capuchon est fixé sur le corps de base, et que le vibreur (15) est en connexion électroconductrice commutable avec la source d'énergie électrique dans le corps de base (2) .

2. Appareil selon la revendication 1, **caractérisé en ce que**, entre le corps de base (2) et le capuchon (3, 9), il y a un premier et un deuxième contact électrique, lesquels se composent respectivement d'un élément de contact de base et d'un élément de contact de capuchon, dans lequel les éléments de contact de base dans le corps de base (2) et les éléments de contact de capuchon dans le capuchon (3) sont disposés de telle sorte que les contacts sont fermés lorsque le capuchon (3) est mis en place sur le corps de base (2), dans lequel la connexion électrique entre la source d'énergie et la deuxième électrode (20) est établie via le premier contact et dans lequel le circuit électrique pour l'alimentation en énergie du vibreur (15) passe par le premier et le deuxième contact.

3. Appareil selon la revendication 2, **caractérisé en ce que** le capuchon (3) en tant que tout, ou une couche extérieure du capuchon (3), se compose d'un matériau électroconducteur, que l'élément de contact de capuchon du premier contact électrique est en connexion avec le matériau de manière électroconductrice et que l'élément de contact de capuchon du deuxième contact électrique est isolé électriquement par rapport au matériau conducteur.

4. Appareil selon l'une des revendications précédentes, **caractérisé en ce que** le vibreur (15) se compose d'un moteur électrique (16) sur l'arbre duquel un excentrique (17) est fixé et que le capuchon présente, sur l'une de ses extrémités, l'extrémité inférieure, une zone de fixation pouvant être fixée sur le corps de base, et forme sur son autre extrémité opposée, l'extrémité supérieure, la deuxième électrode extérieure, et que l'axe du moteur électrique (16) et l'arbre du moteur électrique (16) sont disposés longitudinalement par rapport à une ligne imaginaire entre la zone de fixation et la deuxième électrode extérieure.

5. Appareil selon l'une des revendications 1 à 3, **caractérisé en ce que** le vibreur (15) se compose d'un moteur électrique (16) sur l'arbre duquel un excentrique (17) est fixé et que le capuchon possède sur l'une de ses extrémités, l'extrémité inférieure, une zone de fixation pouvant être fixée sur le corps de base, et forme sur son extrémité opposée, l'extrémité supérieure, la deuxième électrode extérieure, et que l'axe du moteur électrique (16) et l'arbre du moteur électrique (16) sont disposés transversalement à une ligne imaginaire entre la zone de fixation et la deuxième électrode extérieure.

6. Appareil selon l'une des revendications précédentes, **caractérisé en ce que**, dans le capuchon (3), un fond (50) qui enferme une chambre (51) dans la pointe du capuchon est mis en place, lequel présente, sur son dessus tourné vers la chambre (51), un support pour le vibreur (15).

7. Appareil selon la revendication 6, **caractérisé en ce que** deux languettes (53, 54) font saillie à partir du dessous du fond (50) tournant le dos à la chambre (51), dont l'une est dotée d'une fente (26).

8. Appareil selon la revendication 6 ou 7, **caractérisé en ce que** le fond (50) est pénétré par deux broches de contact (18, 19) qui forment les éléments de contact de capuchon, lesquelles font saillie vers le dessus tout comme également le dessous du fond (50), dans lequel l'une des broches de contact (18) est en contact électroconducteur avec la deuxième électrode (20) .

9. Appareil selon la revendication 8, **caractérisé en ce qu'**une prise femelle (65) est formée dans le capuchon (3), dont le côté intérieur est en contact électroconducteur avec la deuxième électrode (20) et qui est étudiée de manière à ce que l'une des broches de contact (18) soit insérée dans la prise femelle (65) lorsque le fond (50) est mis en place dans le capuchon (3).

10. Appareil selon l'une des revendications 6 à 9, **caractérisé en ce que** le fond est pénétré par deux goupilles de fixation (56) qui font saillie vers le dessus, dans lequel des douilles de fixation (62) se trouvent dans le capuchon (3), dans lesquelles les goupilles de fixation (56) sont insérées ou vissées lorsque le fond (50) est mis en place dans le capuchon (3), et que des colonnes (60) partant de la pointe du capuchon sont disposées dans le capuchon (3), dans lequel les douilles de fixation (62) sont réalisées dans les colonnes (60) et leurs ouvertures débouchent dans les côtés frontaux des colonnes (60).

11. Appareil selon l'une des revendications précédentes, **caractérisé en ce qu'**un dispositif de commutation réglable par l'utilisateur de l'appareil est présent dans le corps de base (2), lequel établit les connexions électriques entre la source d'énergie et les électrodes (7, 20), et que le vibreur (15) est également connecté à la source d'énergie électrique via le dispositif de commutation, et que le dispositif de commutation est doté d'une mémoire électronique dans laquelle les paramètres d'état de fonctionnement à sélectionner sont stockés, lesquels peuvent être sélectionnés par l'utilisateur au moyen d'une touche (5), dans lequel l'un des paramètres concerne la polarité des électrodes, et que le vibreur (15) est du genre à fonctionner indépendamment de la polarité de la tension appliquée.

12. Appareil selon l'une des revendications précédentes, **caractérisé en ce que** le capuchon (3, 9) présente, sur son extrémité éloignée du corps de base (2), deux languettes (10, 11) en saillie latérale, lesquelles forment une surface continue pouvant être mise en contact avec la peau à traiter.

13. Appareil selon l'une des revendications précédentes, **caractérisé en ce que** l'on prévoit un dispositif pour détecter une circulation de courant à travers la deuxième électrode, un commutateur dans la connexion entre l'entraînement du vibreur et la batterie et une commande, dans lequel la commande est étudiée de manière à ce que le commutateur est seulement fermé lorsqu'un courant circule à travers la deuxième électrode.

14. Appareil selon l'une des revendications précédentes, **caractérisé en ce qu'**il présente des batteries rechargeables dont les pôles, lors du fonctionnement de l'appareil, sont en connexion électrique avec les électrodes,
et possède un compartiment (72) pour la réception des batteries, lequel est recouvert par un couvercle de boîtier (75) en forme de coque dont le côté extérieur tournant le dos au compartiment (72) forme une partie de la surface extérieure du côté extérieur du boîtier d'appareil,
dans lequel un circuit de charge est prévu, et
que le compartiment (72) est recouvert par un recouvrement de compartiment amovible (77) dont le côté intérieur est orienté vers les batteries (73) dans le compartiment et sur le côté extérieur situé en vis-à-vis duquel est disposée une prise femelle au moins bipolaire, et
que le recouvrement de compartiment (77) se trouve à l'intérieur du couvercle de boîtier (75).
